# EUROPEAN PATENT APPLICATION

(11) **EP 1 911 451 A1**
(43) Date of publication of application: **16.04.2008**
(21) Application number: 06291576.4
(22) Date of filing: 10.10.2006
(51) Int. Cl.: A61K 31/655, A61P 31/12, C09B 56/04

(54) **Protein-kinase CK2 inhibitors and their therapeutic applications**

(71) Applicant: INSERM (Institut National de la Santé et de la Recherche Medicale), 75013 Paris (FR)
(72) Inventor: Cochet, Claude, 38640, Claix (FR); Prudent, Renaud, 38400, Saint Martin D`Heres (FR)
(74) Representative: Colombet, Alain André

(57) **Abstract**

The present invention is related to novel protein-kinase CK2 inhibitors, their method of preparation and their use as anti-cancer, anti-viral or antiinflammatory drugs 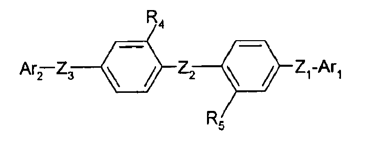 wherein Ar₁, Ar₂, Z₁, Z₂, Z₃, Z₄ and R₅ are as defined in claim 1.

## Description

### Field of the invention

The present invention is related to novel protein-kinase CK2 inhibitors, their method of preparation and their use as anti-cancer or anti-viral drugs.

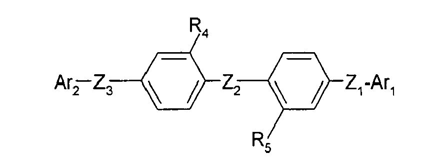

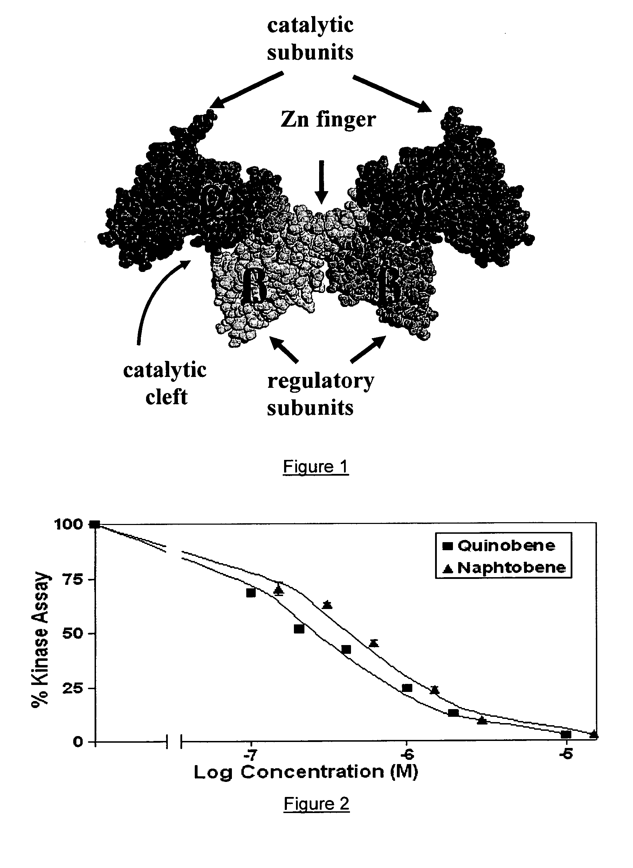

The CK2 protein is an ubiquitous serine/threonine kinase composed of two catalytic subunits α and α' and two identical regulatory β subunits, associated in the formation of αα'β₂, α'₂β₂ or α₂β₂ heterotetramers (Figure 1).

This pleiotropic kinase is essential to embryonic development and to terminal differentiation, and to the control of progression of the cell cycle and of cell survival, and its expression is deregulated in many cancers including tumors of viral origin, where it contributes to the blocking of apoptosis (Buchou et al., 2003; Ahmed et al., 2002).

Because of its essential role in many physiological processes and because of the importance of the pathologies associated with the dysfunction therefore, the CK2 protein represents a new pharmacological target for the development of medicinal products, in particular anticancer and antiviral agents.

Given the lack of physiological devices for turning the CK2 catalytic activity on or off within living cells in conjunction with the lethal consequences of deleting both genes of CK2, catalytic subunits (Padmanabha et al., 1990), makes the use of potent and specific inhibitors the first-choice approach to manipulate this kinase.

However, the development of such molecules has remained limited in the absence of any *in vivo* models for the functional analysis of CK2.

In fact, the few molecules capable of inhibiting CK2 activity that have been described so far have the drawback of being either not very specific or not very active, i.e. :
- several small molecules which are analogs of ATP, capable of specifically inhibiting the catalytic activity of CK2 have been described such as TBB (Sarno et al., 2001), IQA (Sarno et al., 2003) and condensed polyphenolic derivatives (Meggio et al., 2004).
- antisense oligonucleotides directed against the subunits of CK2 (American patent application US 2002/147163 and American patent US 6 455 307 in the name of Isis Pharmaceuticals Inc; Ulloa et al., 1993; Faust et al., 2000); the inhibition of CK2 activity demonstrated only *in vitro* is partial and transient.
- small interfering RNA (siRNA) specific to subunits α, α' and β of CK2 (WO 2005/005632); inhibition of CK2 subunit expression is partial and transient and validation of these siRNA as drugs have not been achieved. Finally, the knockdown of CK2 expression may not be equivalent to inhibition of its catalytic activity and adaptive response might occur.

It emerges from the above that no molecules capable of specifically inhibiting CK2 in an effective manner exists. In addition, no *in vivo* model exists for a functional analysis of CK2, useful for screening for molecules capable of modulating the activity of CK2.

A number of diseases, including cancer, diabetes and inflammation, are linked to perturbation of protein-kinase-mediated cell signaling pathways. Indeed, protein-kinases have proved their remarkable capacity as drug targets in oncology and clinical experience with Gleevec^{™} in chronic myelogenous leukemia and Iressa^{™} and Tarceva^{™} in non-small cell lung cancer has shown that small molecule kinase inhibitors can be therapeutically highly effective (Drucker, 2004).

Thus, there is a need for novel classes of compounds that are both very specific and very active. Surprisingly, the inventors have now discovered a class of compounds that are potent CK2 protein-kinase inhibitors and are useful as anticancer and/or antiviral agents.

The present invention in one aspect is directed to various novel compounds of formula (I): 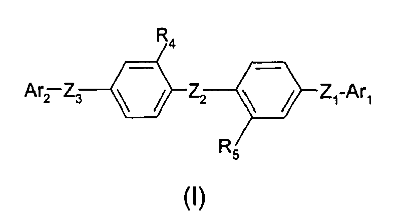 and its stereoisomeric forms, mixtures of stereoisomeric forms, or pharmaceutically acceptable salt forms thereof, wherein the constituent members are defined *infra.*

These compounds are advantageously more efficient than the known CK2 inhibitors. They are notably ATP non competitive, which means that they do not compete with the cellular ATP, thus potentially leading to a high activity at lower therapeutical doses, and consequently with reduced adverse effects.

Further, contrary to TBB (TetraBromoBenzotriazole) and its analogs, the compounds of the invention do not have poly-halogenated aromatic rings, which are likely to display toxicity after metabolization.

Another object of the present invention is to provide pharmaceutical compositions comprising a therapeutically effective amount of at least one compound of formula (I) together with one or more pharmaceutically acceptable excipients and/or carrier.

Another object of the present invention is to provide methods of treating and/or preventing diseases or disorders, including treatment of cancer, viral or inflammatory diseases and disorders.

These and other objects, features and advantages of compounds of formula (I) will be disclosed in the following detailed description of the patent disclosure.

According to a first object, the present invention provides novel compound of formula (I): 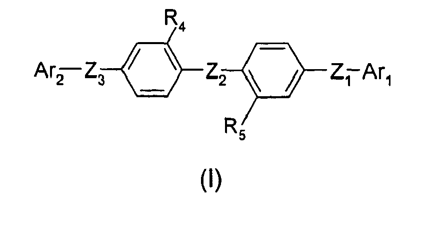 wherein :
Ar₁, Ar₂ are each independently selected from: 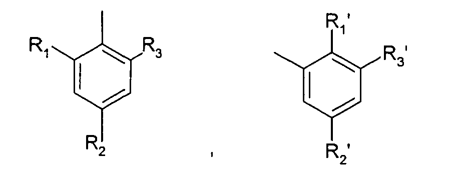 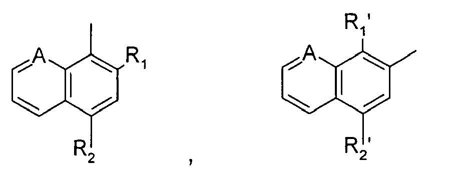 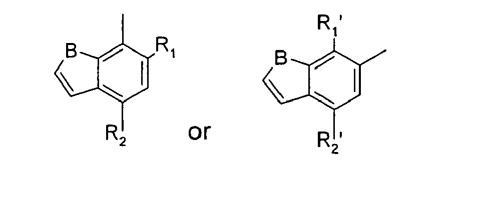 wherein:
A is CH, CR₆ or N;
B is a CH₂, CR₇R₇', O, S(O)_{y}, or NR₈;
R₁, R₁' are each independently selected from H, I, Br, Cl, F, OH, OCOAlk, NH₂, SH, NO₂, or CN;
R₂, R₂', R₄, R₅ are each independently selected from H, I, Br, Cl, F, OH, NO₂, SO₃H, COOH, COOR₉, CONR₁₀R₁₁, (CO)NHOH, or tetrazolyl; R₃, R₃' are each independently selected from H, I, Br, Cl, F, OH, or NH₂;
Z₁, Z₂, Z₃ are each independently selected from -HC=CH-, -X-CH₂-, -CH₂-X-, -N=CH-, -CH=N-, -N=N-, -S-S-, -CO-NH-, -NH-CO-, -CO-CH₂-, -CH₂-CO-, -CO-CH(OH)-, -CH(OH)-CO-, -CH₂-CH(OH)-, -CH(OH)-CH₂-, cyclopropyl, or triazolyl,
X is S, O, CH₂, NH;
y is 0, 1, or 2;
R₆, R₇, R₇', R8, R₉, R₁₀, R₁₁ are each independently selected from H, (C₁-C₆)alkyl, (C₆-C₁₀)aryl(C₁-C₄)alkyl, or (C₆-C₁₀)aryl.
with the exclusion of the compounds wherein:
- A is N, R₁' is OH, R₂' is SO₃H, Z₁ and Z₃ are -N=N-, Z₂ is -HC=CH- and R₄ and R₅ are SO₃H;
- A is CH, R₁' is OH, R₂' is SO₃H, Z₁ and Z₃ are -N=N-, Z₂ is -HC=CH- and R₄ and R₅ are SO₃H;
- A is CH, R₁ is OH, R₂ is H, Z₁ and Z₃ are -N=N-, Z₂ is -HC=CH- and R₄, R₅ are SO₃H;
- A is CH, R₁ is H, R₂ is OH, Z₁ and Z₃ are -N=N-, Z₂ is -HC=CH- and R₄,R₅ are SO₃H;
- A is CH, R₁ is NH₂, R₂ is H, Z₁ and Z₃ are -N=N-, Z₂ is -HC=CH- and R₄, R₅ are SO₃H;
- A is CH, R₁ is H, R₂ is NH₂, Z₁ and Z₃ are -N=N-, Z₂ is -HC=CH- and R₄, R₅ are SO₃H;
- R₁ is H, R₂ is OH, R₃ is H, Z₁ and Z₃ are -N=N-, Z₂ is -HC=CH-, R₄, R₅ are SO₃H;
- R₁ is H, R₂ and R₃ are OH, Z₁ and Z₃ are -N=N-, Z₂ is -HC=CH-, R₄, R₅ are SO₃H;
and the stereoisomeric forms, mixtures of stereoisomeric forms or pharmaceutically acceptable salts forms thereof.

Preferably, Ar₁ and/or Ar₂ are each independently: 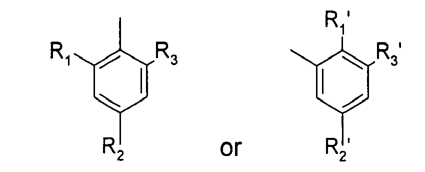 wherein R₁, R₂, R₃, R₁', R₂', R₃' are as defined above.

More preferably, Ar₁ and/or Ar₂ are each independently: 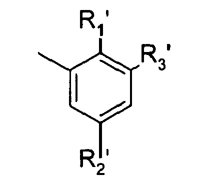 wherein R₁', R₂', R₃' are as defined above.

Preferably, R₁' is NH₂, OH or OCOAlk, most preferably NH₂.

Preferably, R₂' is H, COOH, COOR₉ or SO₃H, wherein R₉ is as defined above, and is notably methyl, ethyl, benzyl.

Preferably, R₃' is H.

Preferably, R₄, R₅ are each independently selected from H or SO₃H. Most preferably, R₄ and/or R₅ is/are SO₃H.

Z₁, Z₂ and/or Z₃ may be a triazolyl, notably:

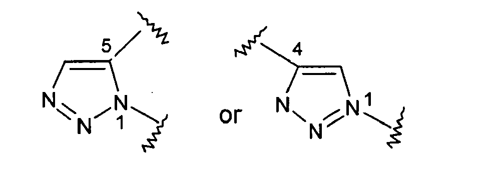

Preferably, Z₁, Z₂ and/or Z₃ are each independently selected from -HC=CH-, -N=N-, -CO-NH-, or -NH-CO-.

Preferably, Z₁ and/or Z₃ are each independently -N=N-, -CO-NH- or -NH-CO-.

Preferably, Z₂ is -HC=CH-, more preferably of *trans* configuration.

According to a particular embodiment, Ar₁, Z₁, R₅ are the same as Ar₂, Z₃ and R₄ respectively.

Preferred compounds may be selected from :

| | Ar₁, Ar₂ | Z₁, Z₃ | Z₂ | R₄, R₅ |
|---|---|---|---|---|
| 1 | 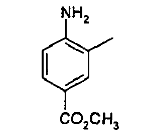 | -N=N- | -HC=CH- (E) | -SO₃H |
| 2 | 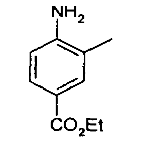 | -N=N- | -HC=CH- (E) | SO₃H |
| 3 | 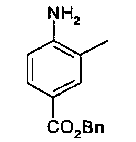 | -N=N- | -HC=CH- (E) | SO₃H |
| 4 | 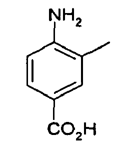 | -N=N- | -HC=CH- (E) | H |
| 5 | 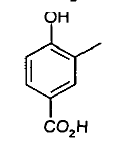 | -N=N- | -HC=CH- (E) | H |
| 6 | 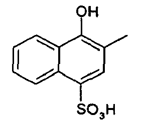 | -N=N- | -HC=CH- (E) | H |
| 7 | 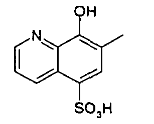 | -N=N- | -HC=CH- (E) | H |
| 8 | 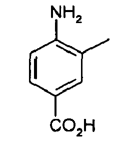 | -CO-NH- | -HC=CH- (E) | SO₃H |
| 9 | 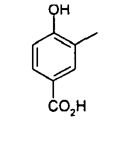 | -CO-NH- | -HC=CH- (E) | SO₃H |
| 10 | 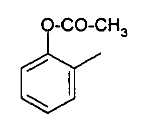 | -CO-NH- | -HC=CH- (E) | SO₃H |
| 11 | 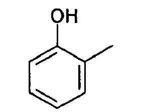 | -CO-NH- | -HC=CH- (E) | SO₃H |
| 12 | 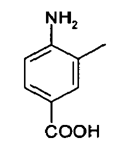 | -CO-NH- | -HC=CH- (E) | H |
| 13 | 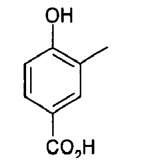 | -CO-NH- | -HC=CH- (E) | H |
| 14 | 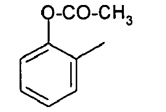 | -CO-NH- | -HC=CH- (E) | H |
| 15 | 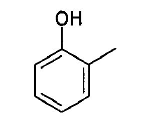 | -CO-NH- | -HC=CH- (E) | H |
| 16 | 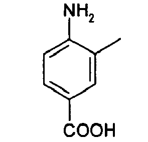 | -N=N- | -HC=CH- (E) | SO₃H |

### Définitions

The following terms and expressions contained herein are defined as follows:

As used herein, the term "Alk" or "alkyl" refers to a straight-chain, or branched alkyl group having 1 to 6 carbon atoms, such as methyl, ethyl, propyl, isopropyl, butyl, isobutyl, sec-butyl, tert-butyl, pentyl, isoamyl, neopentyl, 1-ethylpropyl, 3-methylpentyl, 2,2-dimethylbutyl, 2,3-dimethylbutyl, hexyl. Lower alkyl groups, which are preferred, are alkyl groups as defined above which contain 1 to 4 carbons. A designation such as "C₁-C₄ alkyl" refers to an alkyl radical containing from 1 to 4 carbon atoms.

As used herein, the term "aryl" refers to a mono- or bicyclic hydrocarbon aromatic ring system having 6 to 10 ring carbon atoms. Examples include phenyl and naphthyl. Preferred aryl groups include phenyl and naphthyl groups.

As used herein, the term "arylalkyl" refers to an alkyl group that is substituted with an aryl group, wherein the alkyl and aryl groups are as defined above. Examples of arylalkyl groups include, but are not limited to, benzyl, bromobenzyl, phenethyl, benzhydryl, diphenylmethyl, triphenylmethyl, diphenylethyl, naphthylmethyl, etc.

As used herein, "Hal" means an halogen atom selected from F, Cl, Br or I, preferably from Cl, Br, I.

As used herein, the expression "pharmaceutically acceptable" refers to those compounds, materials, compositions, and/or dosage forms which are, within the scope of sound medical judgment, suitable for contact with the tissues of human beings and animals without excessive toxicity, irritation, allergic response, or other complications commensurate with a reasonable benefit/risk ratio.

All other terms used in the description of the present invention have their meanings as is well known in the art.

In another aspect, the present invention is directed to pharmaceutically acceptable salts of the compounds described above. As used herein, "pharmaceutically acceptable salts" includes salts of compounds of the present invention derived from the combination of such compounds with non-toxic acid or base addition salts.

Acid addition salts include inorganic acids such as hydrochloric, hydrobromic, hydroiodic, sulfuric, nitric and phosphoric acid, as well as organic acids such as acetic, citric, propionic, tartaric, glutamic, salicylic, oxalic, methanesulfonic, para-toluenesulfonic, succinic, and benzoic acid, and related inorganic and organic acids.

Base addition salts include those derived from inorganic bases such as ammonium and alkali and alkaline earth metal hydroxides, carbonates, bicarbonates, and the like, as well as salts derived from basic organic amines such as aliphatic and aromatic amines, aliphatic diamines, hydroxy alkamines, and the like. Such bases useful in preparing the salts of this invention thus include ammonium hydroxide, potassium carbonate, sodium bicarbonate, calcium hydroxide, methylamine, diethylamine, ethylenediamine, cyclohexylamine, ethanolamine and the like.

In addition to pharmaceutically-acceptable salts, other salts are included in the invention. They may serve as intermediates in the purification of the compounds, in the preparation of other salts, or in the identification and characterization of the compounds or intermediates.

The pharmaceutically acceptable salts of compounds of the present invention can also exist as various solvates, such as with water, methanol, ethanol, dimethylformamide, ethyl acetate and the like. Mixtures of such solvates can also be prepared. The source of such solvate can be from the solvent of crystallization, inherent in the solvent of preparation or crystallization, or adventitious to such solvent. Such solvates are within the scope of the present invention.

The present invention also encompasses the pharmaceutically acceptable prodrugs of the compounds disclosed herein. As used herein, "prodrug" is intended to include any compounds which are converted by metabolic processes within the body of a subject to an active agent that has a formula within the scope of the present invention. Since prodrugs are known to enhance numerous desirable qualities of pharmaceuticals (e.g., solubility, bioavailability, manufacturing, etc.) the compounds of the present invention may be delivered in prodrug form. Conventional procedures for the selection and preparation of suitable prodrug derivatives are described, for example, in Prodrugs, Sloane, K. B., Ed.; Marcel Dekker: New York, 1992, incorporated by reference herein in its entirety.

It is recognized that compounds of the present invention may exist in various stereoisomeric forms. As such, the compounds of the present invention notably include geometric isomers of (Z) or (E) configurations and optical isomers.

According to a further object, the present invention is also concerned with the process of preparation of the compounds of formula (I).

The compounds of the present invention may be prepared in a number of ways well known to those skilled in the art. The compounds can be synthesized, for example, by application or adaptation of the methods described below, or variations thereon as appreciated by the skilled artisan. The appropriate modifications and substitutions will be readily apparent and well known or readily obtainable from the scientific literature to those skilled in the art.

In particular, such methods can be found in R.C. Larock, Comprehensive Organic Transformations, VCH publishers, 1989.

It will be appreciated that the compounds of the present invention may contain one or more asymmetrically substituted carbon atoms, and may be isolated in optically active or racemic forms. Thus, all chiral, diastereomeric, racemic forms and all geometric isomeric forms of a structure are intended, unless the specific stereochemistry or isomeric form is specifically indicated. It is well-known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

Compounds of the present invention may be prepared by a variety of synthetic routes. The reagents and starting materials are commercially available, or readily synthesized by well-known techniques by one of ordinary skill in the arts. All substituents, unless otherwise indicated, are as previously defined.

In the reactions described hereinafter, it may be necessary to protect reactive functional groups, for example hydroxy, amino, imino, thio or carboxy groups, where these are desired in the final product, to avoid their unwanted participation in the reactions. Conventional protecting groups may be used in accordance with standard practice, for examples see T.W. Greene et al., 1991; J. F. W. McOmie, 1973.

Some reactions may be carried out in the presence of a base. There is no particular restriction on the nature of the base to be used in this reaction, and any base conventionally used in reactions of this type may equally be used here, provided that it has no adverse effect on other parts of the molecule. Examples of suitable bases include: sodium hydroxide, potassium carbonate, triethylamine, alkali metal hydrides, such as sodium hydride and potassium hydride; alkyllithium compounds, such as methyllithium and butyllithium; and alkali metal alkoxides, such as sodium methoxide and sodium ethoxide.

Usually, reactions are carried out in a suitable solvent. A variety of solvents may be used, provided that it has no adverse effect on the reaction or on the reagents involved. Examples of suitable solvents include: hydrocarbons, which may be aromatic, aliphatic or cycloaliphatic hydrocarbons, such as hexane, cyclohexane, benzene, toluene and xylene; amides, such as dimethylformamide; alcohols such as ethanol and methanol and ethers, such as diethyl ether and tetrahydrofuran.

The reactions can take place over a wide range of temperatures. In general, it is convenient to carry out the reaction at a temperature of from 0°C to 150°C (more preferably from about room temperature to 100°C). The time required for the reaction may also vary widely, depending on many factors, notably the reaction temperature and the nature of the reagents. However, provided that the reaction is effected under the preferred conditions outlined above, a period of from 3 hours to 20 hours will usually suffice.

The compound thus prepared may be recovered from the reaction mixture by conventional means. For example, the compounds may be recovered by distilling off the solvent from the reaction mixture or, if necessary, after distilling off the solvent from the reaction mixture, pouring the residue into water followed by extraction with a water-immiscible organic solvent and distilling off the solvent from the extract. Additionally, the product can, if desired, be further purified by various well-known techniques, such as recrystallization, reprecipitation or the various chromatography techniques, notably column chromatography or preparative thin layer chromatography.

The process of preparation of compounds of formula (I) of the invention is another object of the present invention.

According to a first aspect, compounds of the invention of formula (I), wherein Z₁ and/or Z₃ are/is -NH-CO, can be obtained from a compound of formula (II) 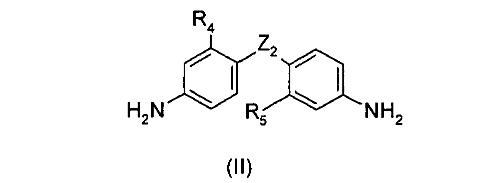 wherein R₄, R₅, Z₂ are as defined above.

Generally, a reaction comprising the step of coupling said compound of formula (II) with a compound Ar₁-CO-Hal, Ar₁-COOH, Ar₁-COOAlk, and/or Ar₂-CO-Hal, Ar₂-COOH, Ar₂-COOAlk, in which Ar₁, Ar₂ are as defined above, is carried out.

According to a further aspect, the compounds of formula (I), wherein Z₁ and/or Z₃ are/is -S-S-, can be obtained from a compound of formula (III) : 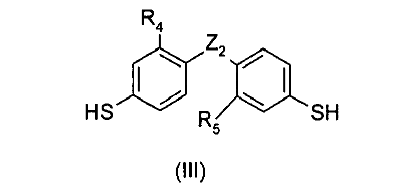 wherein R₄, R₅, Z₂ are as defined above.

Generally, a reaction comprising the step of coupling Ar₁SH and/or Ar₂SH, in which Ar₁, Ar₂ are as defined above, is carried out.

According to a further aspect, the compounds of formula (I), wherein Z₁ and/or Z₃ are/is -HC=CH-, can be obtained from a compound of formula (IV): 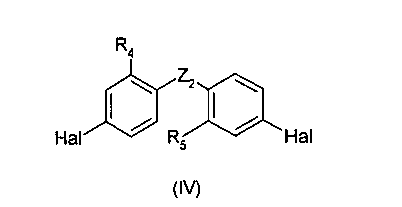 wherein R₄, R₅, Hal, Ar₁ and Ar₂ are as defined above.

Generally, a reaction comprising an organometallic coupling with Ar₁-CH=CH₂ and Ar₂-CH=CH₂, is carried out.

Alternatively, according to a further aspect, the compounds of formula (I), wherein Z₁ and/or Z₃ are/is -HC=CH-, can be obtained from a compound of formula (V): 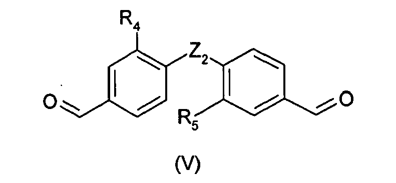 wherein R₄, R₅, Z₂ are as defined above.

Generally, a reaction comprising a Wittig reaction with Ar₁-CH₂-P(Alk)₃ or Ar₁-CH₂-P(Aryl)₃, wherein Ar₁, Ar₂ are as defined above, is carried out.

According to a further aspect, the compounds of formula (I), wherein Z₁ and/or Z₃ are/is triazolyl, can be obtained from a compound of formula (VI): 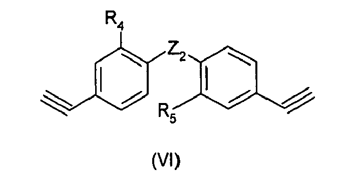 wherein R₄, Z₂ are as defined above.

Generally, a reaction comprising the step of coupling a compound Ar₁-N₃ and/or Ar₂-N₃ with a compound of formula (VI) according to a Huisgen reaction is carried out.

Similarly, compounds of formulae (II) to (VI), wherein Z₂ is -NH-CO-, -S-S, -HC=CH- or triazolyl, may be obtained according to the reactions disclose above.

Further, the process of the invention may also comprise the additional step of isolating the compound of formula (I). This can be done by the skilled person by any of the known conventional means, such as the recovery methods described above.

The starting products are commercially available or may be obtained by applying or adapting any known methods or those described in the examples.

According to a further object, the present invention is also concerned with pharmaceutical compositions comprising a compound of formula (I) together with a pharmaceutically acceptable excipient or carrier.

The CK2 protein-kinase inhibitors are advantageously used as active ingredient, in particular, of anticancer, antiviral or anti-inflammatory medicaments.

Preferably, said compounds may be used to treat and/or prevent the cancers selected from breast, prostate, squamous cell carcinoma of head and neck, lung, brain or glioma cancer.

Alternatively, said compounds may be used to prevent and/or to treat viral diseases and disorders notably selected from Herpes, Cytomegalovirus, or inflammatory diseases and disorders, notably chronic inflammatory disorders (in particular chronic intestinal inflammation), or glomerulonephritis.

According to a further object, the present invention concerns the use of a compound of formula (I): 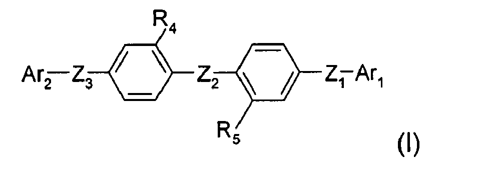 wherein :
Ar₁, Ar₂ are each independently selected from: 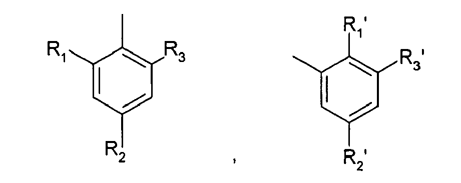 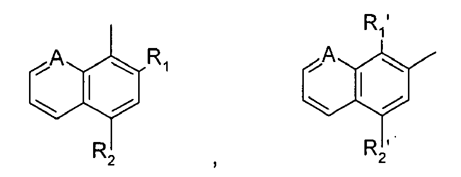 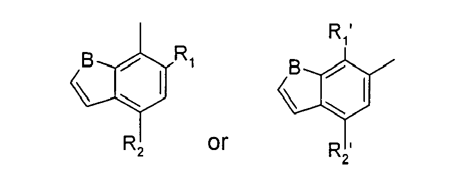
wherein:
A is CH, CR₆ or N;
B is a CH₂, CR₇R₇', O, S(O)_{y}, or NR₈;
R₁, R₁' are each independently selected from H, I, Br, Cl, F, OH, OCOAlk, NH₂, SH, NO₂, or CN;
R₂, R₂', R₄, R₅ are each independently selected from H, I, Br, Cl, F, OH, NO₂, SO₃H, COOH, COOR₉, CONR₁₀R₁₁, (CO)NHOH, or tetrazolyl; R₃, R₃' are each independently selected from H, I, Br, Cl, F, OH, or NH₂;
Z₁, Z₂, Z₃ are each independently selected from -HC=CH-, -X-CH₂-, -CH₂-X-, -N=CH-, -CH=N-, -N=N-, -S-S-, -CO-NH-, -NH-CO-, -CO-CH₂-, -CH₂-CO-, -CO-CH(OH)-, -CH(OH)-CO-, -CH₂-CH(OH)-, -CH(OH)-CH₂-, cyclopropyl, or triazolyl,
X is S, O, CH₂, NH;
y is 0, 1, or 2;
R₆, R₇, R₇', R₈, R₉, R₁₀, R₁₁ are each independently selected from H, (C₁-C₆)alkyl, (C₆-C₁₀)aryl(C₁-C₄)alkyl, or (C₆-C₁₀)aryl.
and the stereoisomeric forms, mixtures of stereoisomeric forms or pharmaceutically acceptable salts forms thereof,
for the preparation of a medicament for inhibiting protein-kinase CK2.

In particular, the CK2 protein-kinase inhibitors may be used for the treatment and/or prevention of pathology associated with a deregulated (i.e. increased or decreased) activity of CK2 in cells.

According to a still further object, the present invention is also concerned with the use of a compound of formula (I) for the preparation of a medicament intended for the prevention and/or treatment of cancer, viral or inflammatory diseases and disorders.

Said cancer may be selected from the group consisting of breast, prostate, squamous cell carcinoma of head and neck, lung, brain or glioma cancer.

The present invention also concerns the corresponding methods of treatment comprising the administration of a therapeutically effective amount of a compound of the invention together with a pharmaceutically acceptable carrier or excipient to a patient in the need thereof.

The identification of those subjects who are in need of treatment of herein-described diseases and conditions is well within the ability and knowledge of one skilled in the art. A clinician skilled in the art can readily identify, by the use of clinical tests, physical examination and medical/family history, those subjects who are in need of such treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

The amount of a compound of formula (I), which is required to achieve the desired biological effect, will vary depending upon a number of factors, including the dosage of the drug to be administered, the chemical characteristics (e.g. hydrophobicity) of the compounds employed, the potency of the compounds, the type of disease, the diseased state of the patient and the route of administration.

As used herein, "pharmaceutically acceptable carrier" includes any diluents, adjuvants, excipients, or vehicles, such as preserving agents, fillers, disintegrating agents, wetting agents, emulsifying agents, suspending agents, solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

In the context of the invention, the term "treating" or "treatment", as used herein, means reversing, alleviating, inhibiting the progress of, or preventing the disorder or condition to which such term applies, or one or more symptoms of such disorder or condition.

"Therapeutically effective amount" means an amount of a compound/medicament according to the present invention effective in producing the desired therapeutic effect.

According to the invention, the term "patient", or "patient in need thereof", is intended for a human or non-human mammal affected or likely to be affected with a disease or disorder disclosed therein. Preferably, the patient is a human.

The invention is further illustrated but not restricted by the description in the following examples.

### Figures

Figure 1: CK2 is a macromolecular complex made by the reversible association of two catalytic subunits with two regulatory subunits.
Figure 2: Inhibition of the catalytic activity of recombinant CK2α by Quinobene (IC₅₀: 0.13 µM) or Naphtobene (IC₅₀: 0.30 µM).
Figure 3: Quinobene is a non-competitive inhibitor of CK2α.
   Lineweaver-Burk inhibition plots of CK2α in the presence of different concentrations of Quinobene (0 ; 0.1 ; 0.2 ; 0.4 ; 0.8 µM).
   (A) variable concentrations of ATP with Peptide] = 500 µM. (B) variable concentrations of Peptide with [ATP] = 200 µM. Similar results are obtained with Naphtobene.
Figure 4: Inhibition of the catalytic activity of recombinant CK2α by CCB8 (IC₅₀: 0.6 µM).

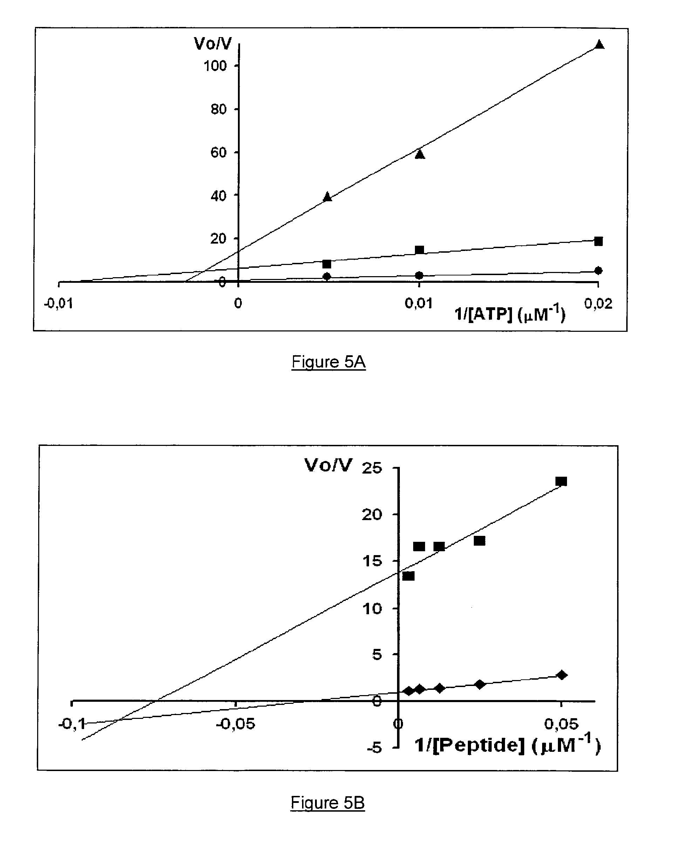

Figure 5: CCB8 is a non-competitive inhibitor of CK2α.
   Lineweaver-Burk inhibition plots of CK2α in the presence of different concentrations of CCB8 (0 ; 1.1 ; 2.2 µM for ATP competition assay; 0 ; 1.1 µM for peptide competition assay). (A) variable concentrations of ATP with [Peptide] = 166 µM. (B) variable concentrations of Peptide with [ATP] = 25 µM. Ki constant deduced from this graph is 85 nM.

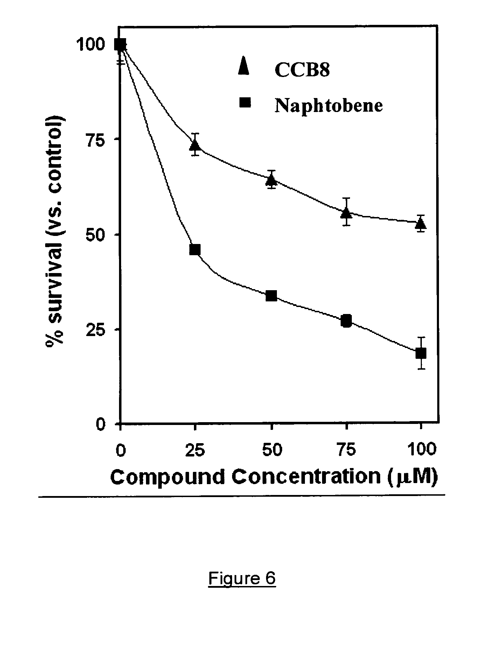

Figure 6: Effects of CCB8 or Naphtobene on cancer cell viability.
   A549 cells were treated for 48 hours with increasing concentrations of CCB8 (σ) or Naphtobene (ν). Cell viability was evaluated with the Cell Titer Glo kit (Promega).

### Examples

¹H NMR analysis were performed on Brucker Spectrospin 300 MHz.

MS data were collected on Brucker LC-MS-MS (electrospray negative mode).

Thin Layer Chromatography (TLC) was performed on plastic sheets silica gel 60 F₂₅₄ thickness 0.2 mm, Merck.

### Example 1

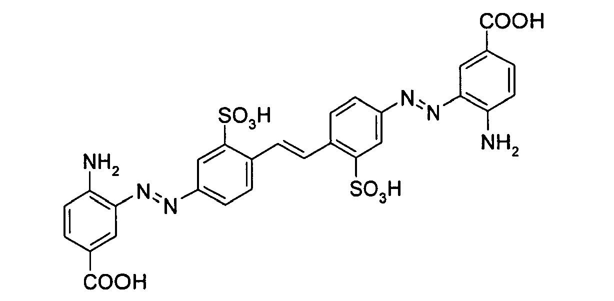

Compound (16) referred to herein as CCB8.

This synthesis was adapted from Gruszecka-Kowalik E. et al., 1992.

### Preparation of solution A

4,4'-diamino-2,2'-stilbene disulfonic acid (1.765 mmol, 653 mg, Sigma) was added to 2.65 mL of MilliQ water under stirring. 176.5 µL HCl were added. The temperature was then set up at 50°C for 10 minutes and cooled down to 10°C. 185 µL HCl were added. 353 µL of an extemporaneously made solution of sodium nitrite (1g NaNO₂ in 2.84 mL milliQ water) were added. The reaction was stirred for 30 minutes at 10°C.

### Preparation of solution B

In the meantime, 4-aminobenzoic acid (1.575 mmol, 219 mg, Merck) was dissolved in 5.4 mL MilliQ water under stirring. 232 µL of a solution of sodium hydroxide (1.2 g NaOH in 4.5 mL milliQ water) were added, followed by 0.3 mL of a solution of sodium carbonate (2.702 g anhydrous Na₂CO₃ in 3 mL MilliQ water). The resulting solution was then cooled down to 10°C under vigorous stirring.

Then 847 µL of solution B were added to 210 µL of solution A. The resulting mixture was stirred for 2 hours at 4°C, then, 5 hours at room temperature. The reaction mixture was dried over-night under vacuum to give CCB8 as a brown crude product.

### Purification of the crude product

The crude product was then purified by the following procedure:

72 mg was dissolved in 0.5 mL MilliQ water and purified by semi-preparative chromatography on TLC silica sheets (TLC plastic sheets silica gel 60 F₂₅₄ thickness 0.2 mm, Merck) eluted by Isopropanol/EtOH/H₂O/NH₄OH 10:10:2:1. After air-drying, the non-migrating product was recovered from the silica plate by scrapping the corresponding spot and suspended in water. After vigorous shaking and centrifugation at 14.000 rpm for 10 minutes at room temperature, the resulting supernatant was dried under vacuum.

The product was then re-chromatographied using EtOH/H₂O/NH₄OH 14:6:1 as an eluant. The CCB8 product with Rf ~ 0.52 was recovered as described above.

Final purification was performed by HPLC using a C18 column (Inertsil ODS-2 C18 5 µM particle 150 mm * 4 mm) in 25 mM ammonium formiate (pH~5) with 0 to 40% MeCN gradient over 30 minutes at a flow rate of 1 mL.min⁻¹ at room temperature. Due to its sulfonated groups, CCB8 eluted as a broad peak with a retention time tᵣ=16min. As previously mentioned in Naicker et al. (Bioorg. & Med. Chem. 12 (2004) 1215-1220), this kind of product is difficult to purify and requires repeated chromatography.
¹H NMR (δ, ppm) : (CDCl₃)
6.74 (d, J=8Hz), 7.66 (D, J=8Hz); 6.85 (arom); 7.27 (arom); 7.72 (arom); 7.85 (arom); 7.91 (arom).
MS : [M-H]⁻ was 665,3Da versus 665Da calculated for CCB8.

### Example 2 : CK2 protein-kinase inhibition

### A- Quinobene and Naphtobene

4,4'-bis(8-hydroxy-5-sulfo-7-quinolineazo)stilbene-2,2'-disulfonic acid (Quinobene) or 4,4'-[(1-hydroxy-4-sulfo-2-naphtyl)azo] 2,2'-stilbenedisulfonic acid (Naphtobene) were obtained from the National Cancer Institute and screened for CK2 inhibitors using an *in vitro* CK2 kinase assay (Filhol et al., 1991).

These compounds inhibited CK2 catalytic activity in a dose-dependent manner (the half-maximal effective concentration is 0.13 and 0.30 µM for Quinobene and Naphtobene respectively) (Figure 2).

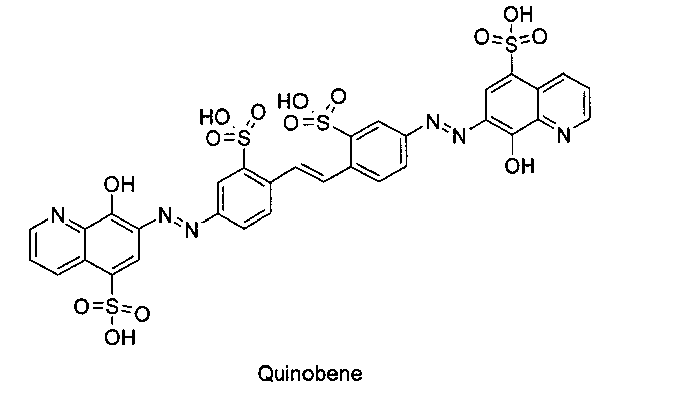

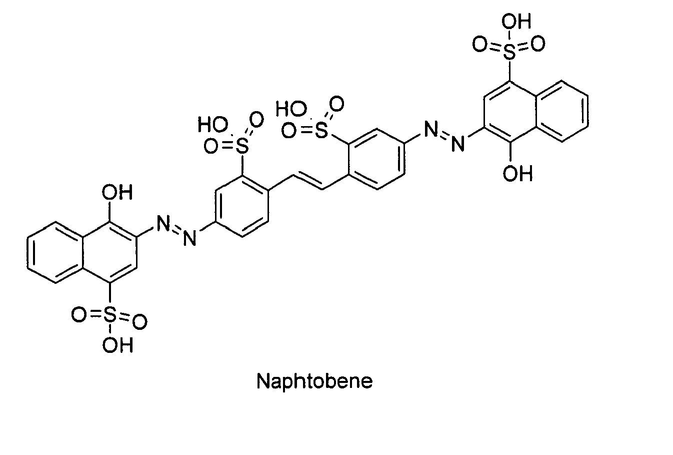

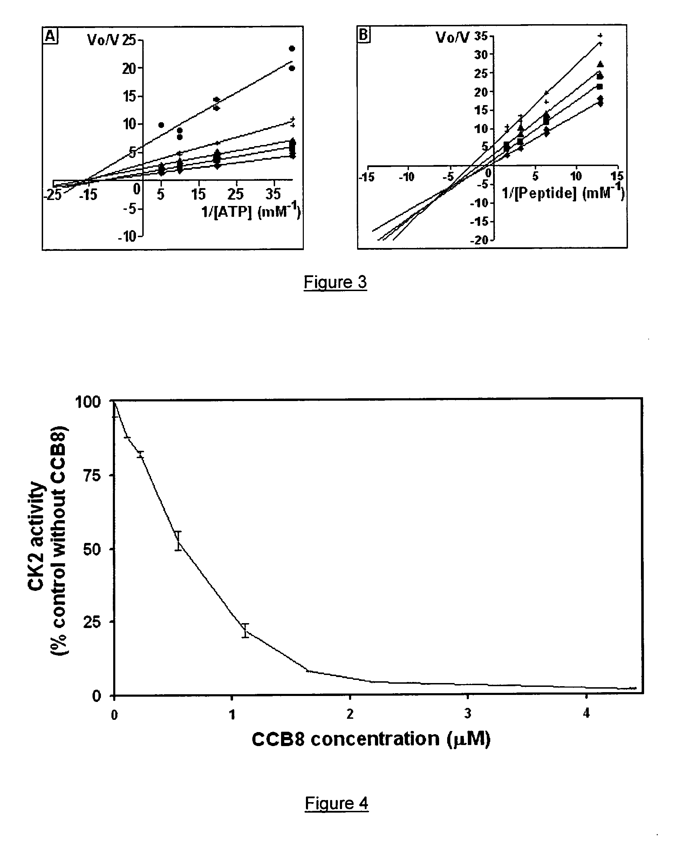

These new inhibitors were shown to be non-competitive with respect to ATP or the phosphoacceptor substrate. Invariably, the inhibitors do not increase the Km for ATP or for the phosphoacceptor peptide (Figure 3).

To check if Quinobene which efficiently inhibits CK2 *in vitro* is also efficient on it *in vivo,* non-small cell lung cancer cells (A549) were treated with 100 µM Naphtobene for 48 hours. A confocal microscopic analysis showed that Naphtobene is cell permeable accumulating in the cytoplasmic compartment.

A549 cells were treated with increasing concentrations of TBB 4,5,6,7-TetraBromoBenzotriazole (Calbiochem®) or Quinobene for 48 hours and the cell viability was monitored. The data indicate that Quinobene potency correlates with its proapoptotic efficacy (Figure 6).

### B- CCB8

CCB8 was tested in the *in vitro* CK2 assay.

CCB8 appears to be as potent as Quinobene (IC₅₀ = 0.6 µM), but smaller and more hydrophilic (Figure 4).

Like Quinobene, CCB8 behaves as a non-competitive inhibitor toward ATP or the phosphoacceptor peptide (Figure 6).

To check if CCB8 which like Naphtobene efficiently inhibits CK2 *in vitro* is also efficient on it *in vivo,* non-small cell lung cancer cells (A549) were treated with increasing concentrations of CCB8 or Naphtobene for 48 hours and the cell viability was monitored.

The data indicate that like for Naphtobene, the CCB8 potency correlates with its proapoptotic efficacy (Figure 6).

### References

The following references, to the extent that they provide exemplary procedural or other details supplementary to those set forth herein, are specifically incorporated in their entirety herein by reference:
- Ahmed K., Gerber D.A. and Cochet C., 2002, Joining the cell survival squad: an emerging role for protein-kinase CK2. Trends Cell Biol., 12, 226-230
- Buchou T. and Cochet C., 2003, La protéine kinase CK2 : une enzyme qui cultive la difference, Medecine/Sciences, 19, 709-716
- Drucker, 2004, Oncologist, 9, 357-360
- Faust R.A., Tawfic S., Davis A.T., Bubash L.A., Ahmed K., 2000, Antisense oligonucleotide against protein-kinase CK2-alpha inhibit growth of squamous cell carcinoma of the head and neck in vitro. Head Neck. 22(4):341-6
- Filhol O., Cochet C., Wedegaertner P., Gill G.N., Chambaz E.M., 1991, Coexpression of both α and β subunits is required for assembly of regulated Casein Kinase II. Biochemistry. 30, 1133-11140.
- Gruszecka-Kowalik E., Haugwitz R.D., Zalkow L.H., "Quinobene, a new potent anti-HIV agent", 1992, BBRC, 187, 1409-17
- J. F. W. McOmie in Protective Groups in Organic Chemistry, Plenum Press, 1973
- Meggio F., Pagano M.A., Moro S., Zagotto G., Ruzzene M., Sarno S., Cozza G., Bain J., Elliott M., Donella-Deana A., Brunati A.M., Pinna L.A., 2004, Inhibition of Protein-kinase CK2 by condensed polyphenolic derivatives. An in vitro and in vivo study. Biochemistry, 43, 12931-12936
- Naicker et al., Bioorg. & Med. Chem. 12 (2004) 1215-1220
- Padmanabha R., Chen Wu J.L., Hanna D.E. and Glover C.V.C., 1990, Isolation, sequencing and disruption of the yeast CKA2 gene: casein kinase II is essential for viability in Saccharomyces cerevisiae. Mol Cell Biol., 10, 4089-4099
- Parhar K., Morse J., Salh B. 2006, The role of protein-kinase CK2 in intestinal epithelial cell inflammatory signalling. Int J. Colorectal Dis. (in press)
- Sarno S., Reddy H., Meggio F., Ruzzene M., Davies S.P., Donella-Deana A., Shugar D., Pinna L.A., 2001, Selectivity of 4,5,6,7-tetrabromobenzotriazole, an ATP site-directed inhibitor of protein-kinase CK2. FEBS Letters, 496, 44-48
- Ulloa L., Diaz-Nido J., Avila J., 1993, Depletion of casein kinase II by antisense oligonucleotide privent neuritogenesis in neuroblastoma cells, EMBO J. 4, 1633-40
- US 2002/147163 and US 6 455 307 in the name of Isis Pharmaceuticals Inc
- W. Greene and P. G. M. Wuts in Protective Groups in Organic Chemistry, John Wiley and Sons, 1991
- WO 2005/005632

## Claims

1. A compound of formula (I): 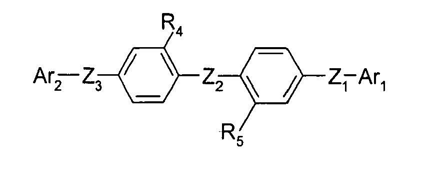 wherein :
Ar₁, Ar₂ are each independently selected from: 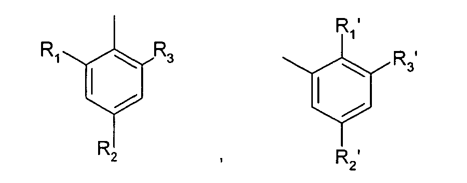 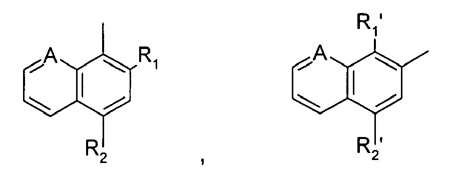 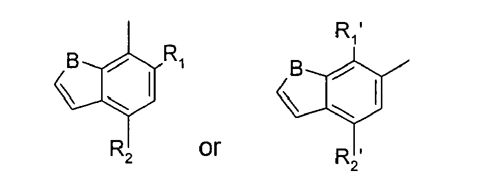
wherein:
A is CH, CR₆ or N;
B is a CH₂, CR₇R₇', O, S(O)_{y}, or NR₈;
R₁, R₁' are each independently selected from H, I, Br, Cl, F, OH, OCOAlk, NH₂, SH, NO₂, or CN;
R₂, R₂', R₄, R₅ are each independently selected from H, I, Br, Cl, F, OH, NO₂, SO₃H, COOH, COOR₉, CONR₁₀R₁₁, (CO)NHOH, or tetrazolyl;
R₃, R₃' are each independently selected from H, I, Br, Cl, F, OH, or NH₂;
Z₁, Z₂, Z₃ are each independently selected from -HC=CH-, -X-CH₂-, -CH₂-X-, -N=CH-, -CH=N-, -N=N-, -S-S-, -CO-NH-, -NH-CO-, -CO-CH₂-, -CH₂-CO-, -CO-CH(OH)-, -CH(OH)-CO-, -CH₂-CH(OH)-, -CH(OH)-CH₂-, cyclopropyl, or triazolyl,
X is S, O, CH₂, NH;
y is 0, 1, or 2;
R₆, R₇, R₇', R₈, R₉, R₁₀, R₁₁ are each independently selected from H, (C₁-C₆)alkyl, (C₆-C₁₀)aryl(C₁-C₄)alkyl, or (C₆-C₁₀)aryl.
with the exclusion of the compounds wherein:
- A is N, R₁' is OH, R₂' is SO₃H, Z₁ and Z₃ are -N=N-, Z₂ is -HC=CH- and R₄ and R₅ are SO₃H;
- A is CH, R₁' is OH, R₂' is SO₃H, Z₁ and Z₃ are -N=N-, Z₂ is -HC=CH- and R₄ and R₅ are SO₃H;
- A is CH, R₁ is OH, R₂ is H, Z₁ and Z₃ are -N=N-, Z₂ is -HC=CH- and R₄, R₅ are SO₃H;
- A is CH, R₁ is H, R₂ is OH, Z₁ and Z₃ are -N=N-, Z₂ is -HC=CH- and R₄,R₅ are SO₃H;
- A is CH, R₁ is NH₂, R₂ is H, Z₁ and Z₃ are -N=N-, Z₂ is -HC=CH- and R₄, R₅ are SO₃H;
- A is CH, R₁ is H, R₂ is NH₂, Z₁ and Z₃ are -N=N-, Z₂ is -HC=CH- and R₄, R₅ are SO₃H;
- R₁ is H, R₂ is OH, R₃ is H, Z₁ and Z₃ are -N=N-, Z₂ is -HC=CH-, R₄, R₅ are SO₃H;
- R₁ is H, R₂ and R₃ are OH, Z₁ and Z₃ are -N=N-, Z₂ is -HC=CH-, R₄, R₅ are SO₃H;
and the stereoisomeric forms, mixtures of stereoisomeric forms or pharmaceutically acceptable salts forms thereof.

2. The compound according to claim 1, wherein Ar₁ and/or Ar₂ are each independently 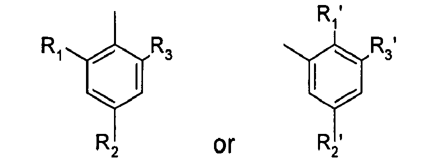 wherein R₁, R₂, R₃, R₁', R₂', R₃' are as defined in claim 1.

3. The compound of claim 2, wherein Ar₁ and/or Ar₂ are each independently 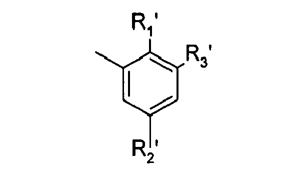 wherein R₁', R₂', R₃' are as defined in claim 1.

4. The compound according to claims 1 to 3, wherein R₁' is NH₂, OH or OCOAIk.

5. The compound according to any of claims 1 to 4, wherein R₂' is H, COOH, COOR₉ or SO₃H, wherein R₉ is as defined in claim 1.

6. The compound according to any of claims 1 to 5, wherein R₃' is H.

7. The compound according to any of claims 1 to 6, wherein R₄ and/or R₅ is/are H or SO₃H.

8. The compound according to any of claims 1 to 7, wherein Z₁, Z₂ and/or Z₃ are each independently selected from -HC=CH-, -N=N-, -CO-NH-, or -NH-CO-.

9. The compound according to claim 8, wherein Z₁ and/or Z₃ are each independently -N=N-, -CO-NH- or -NH-CO-.

10. The compound according to claim 8, wherein Z₂ is -HC=CH-.

11. The compound according to claim 10, wherein -HC=CH- is *trans.*

12. The compound according to any of claims 1 to 11, which is selected from:
| | | | | |
|---|---|---|---|---|
| | Ar₁, Ar₂ | Z₁, Z₃ | Z₂ | R₄, R₅ |
| 1 | 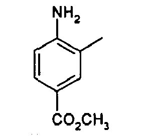 | -N=N- | -HC=CH- (E) | -SO₃H |
| 2 | 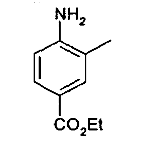 | -N=N- | -HC=CH- (E) | SO₃H |
| 3 | 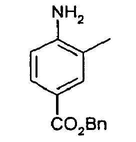 | -N=N- | -HC=CH- (E) | SO₃H |
| 4 | 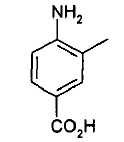 | -N=N- | -HC=CH- (E) | H |
| 5 | 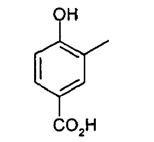 | -N=N- | -HC=CH- (E) | H |
| 6 | 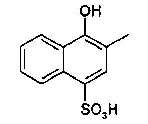 | -N=N- | -HC=CH- (E) | H |
| 7 | 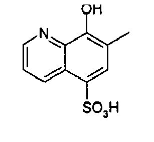 | -N=N- | -HC=CH- (E) | H |
| 8 | 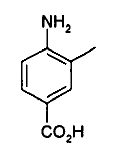 | -CO-NH- | -HC=CH- (E) | SO₃H |
| 9 | 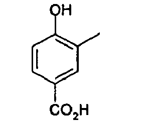 | -CO-NH- | -HC=CH- (E) | SO₃H |
| 10 | 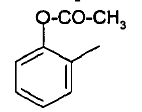 | -CO-NH- | -HC=CH- (E) | SO₃H |
| 11 | 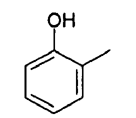 | -CO-NH- | -HC=CH- (E) | SO₃H |
| 12 | 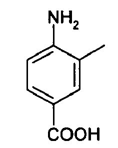 | -CO-NH- | -HC=CH- (E) | H |
| 13 | 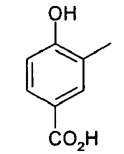 | -CO-NH- | -HC=CH- (E) | H |
| 14 | 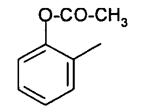 | -CO-NH- | -HC=CH- (E) | H |
| 15 | 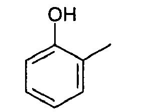 | -CO-NH- | -HC=CH (E) | H |
| 16 | 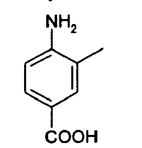 | -N=N- | - -HC=CH- (E) | H SO₃H |

13. A pharmaceutical composition comprising a compound of any of claims 1 to 12 together with a pharmaceutically acceptable excipient or carrier.

14. A use of a compound of formula (I): 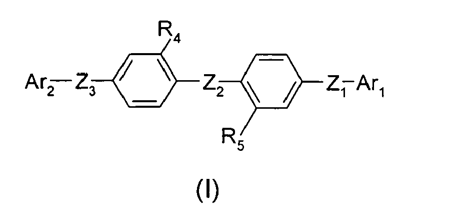 wherein :
Ar₁, Ar₂ are each independently selected from: 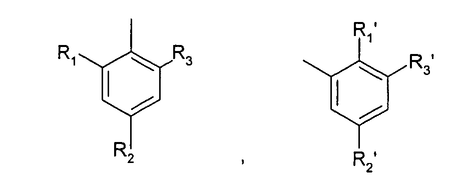 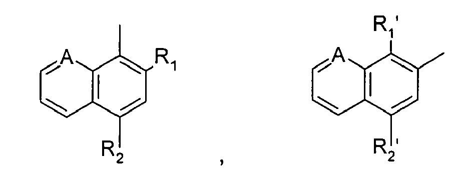 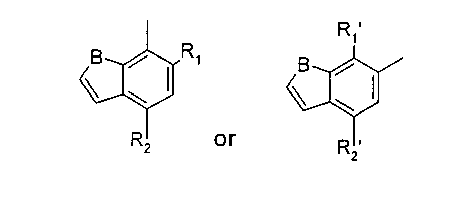 wherein:
A is CH, CR₆ or N;
B is a CH₂, CR₇R₇', O, S(O)_{y}, or NR₈;
R₁, R₁' are each independently selected from H, I, Br, Cl, F, OH, OCOAIk, NH₂, SH, NO₂, or CN;
R₂, R₂', R₄, R₅ are each independently selected from H, I, Br, Cl, F, OH, NO₂, SO₃H, COOH, COOR₉, CONR₁₀R₁₁, (CO)NHOH, or tetrazolyl; R₃, R₃' are each independently selected from H, I, Br, Cl, F, OH, or NH₂;
Z₁, Z₂, Z₃ are each independently selected from -HC=CH-, -X-CH₂-, -CH₂-X-, -N=CH-, -CH=N-, -N=N-, -S-S-, -CO-NH-, -NH-CO-, -CO-CH₂-, -CH₂-CO-, -CO-CH(OH)-, -CH(OH)-CO-, -CH₂-CH(OH)-, -CH(OH)-CH₂-, cyclopropyl, or triazolyl,
X is S, O, CH₂, NH;
y is 0, 1, or 2;
R₆, R₇, R₇', R₈, R₉, R₁₀, R₁₁ are each independently selected from H, (C₁-C₆)alkyl, (C₆-C₁₀)aryl(C₁-C₄)alkyl, or (C₆-C₁₀)aryl.
and the stereoisomeric forms, mixtures of stereoisomeric forms or pharmaceutically acceptable salts forms thereof,
for the preparation of a medicament for inhibiting protein-kinase CK2.

15. A use of a compound of formula (I): 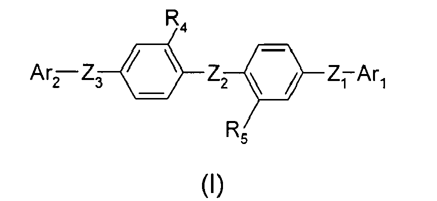 wherein :
Ar₁, Ar₂ are each independently selected from: 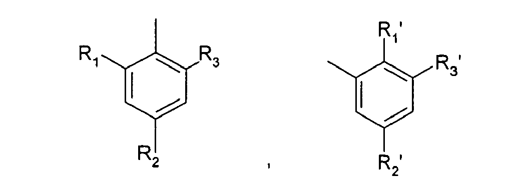 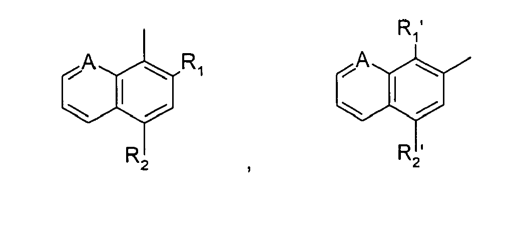 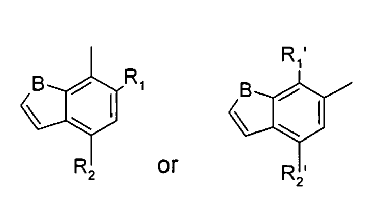 wherein:
A is CH, CR₆ or N;
B is a CH₂, CR₇R₇', O, S(O)_{y}, or NR₈;
R₁, R₁' are each independently selected from H, I, Br, Cl, F, OH, OCOAlk, NH₂, SH, NO₂, or CN;
R₂, R₂', R₄, R₅ are each independently selected from H, I, Br, Cl, F, OH, NO₂, SO₃H, COOH, COOR₉, CONR₁₀R₁₁, (CO)NHOH, or tetrazolyl; R₃, R₃' are each independently selected from H, I, Br, Cl, F, OH, or NH₂;
Z₁, Z₂, Z₃ are each independently selected from -HC=CH-, -X-CH₂-, -CH₂-X-, -N=CH-, -CH=N-, -N=N-, -S-S-, -CO-NH-, -NH-CO-, -CO-CH₂-, -CH₂-CO-, -CO-CH(OH)-, -CH(OH)-CO-, -CH₂-CH(OH)-, -CH(OH)-CH₂-, cyclopropyl, or triazolyl,
X is S, O, CH₂, NH;
y is 0, 1, or 2;
R₆, R₇, R₇', R₈, R₉, R₁₀, R₁₁ are each independently selected from H, (C₁-C₆)alkyl, (C₆-C₁₀)aryl(C₁-C₄)alkyl, or (C₆-C₁₀)aryl,
and the stereoisomeric forms, mixtures of stereoisomeric forms or pharmaceutically acceptable salts forms thereof, for the preparation of a medicament intended for the treatment and/or prevention of cancer, viral or inflammatory diseases and disorders.

16. A use of a compound according to any of claims 1 to 12 for the preparation of a medicament intended for the prevention and/or treatment of cancer, viral or inflammatory diseases and disorders.

17. The use according to claim 15 or 16, wherein said cancer is selected from breast, prostate, lung, brain, glioma cancer or squamous cell carcinoma of head and neck.

18. The use of a compound according to claim 15 or 16, wherein said viral diseases and disorders are selected from Herpes and Cytomegalovirus.

19. The use of a compound according to claim 15 or 16, wherein said inflammatory diseases and disorders are selected from chronic inflammatory disorders or glomerulonephritis.
